(19) Europäisches Patentamt
European Patent Office
Office européen des brevets

(11) Publication number: 0 448 099 A2

(12) EUROPEAN PATENT APPLICATION

(21) Application number: 91104440.2

(51) Int. Cl.5: C07K 7/08, A61K 37/02

(22) Date of filing: 21.03.91

(30) Priority: 23.03.90 JP 73895/90

(43) Date of publication of application: 25.09.91 Bulletin 91/39

(84) Designated Contracting States: CH DE DK ES FR GB IT LI NL SE

(71) Applicant: KUREHA CHEMICAL INDUSTRY CO., LTD.
9-11, Horidome-cho, 1-chome Nihonbashi
Chuo-ku Tokyo 103(JP)

(72) Inventor: Chiba, Yukinobu
Kureha-Kagaku Nerima-so, 106,10-13, Nerima 3-chome
Nerima-ku, Tokyo 176(JP)

(74) Representative: Vossius & Partner
Siebertstrasse 4 P.O. Box 86 07 67
W-8000 München 86(DE)

# (54) Novel peptides.

(57) Peptides are disclosed which have the general formula (I)

$$R_1\text{-Ser-Lys-Leu-Asn-Asp-Arg-Ala-Asp-Ser-Arg-Arg-Ser-Leu-Trp-Asp-}R_2 \quad (I)$$

wherein $R_1$ is H-, H-Pro-, H-Gly-Pro, H-Lys-Gly-Pro-, H-Thr-Lys-Gly-Pro-, H-Leu-Thr-Lys-Gly-Pro-, H-Phe-Leu-Thr-Lys-Gly-Pro-, H-Ser-Phe-Leu-Thr-Lys-Gly-Pro-,
H-Gly-Ser-Phe-Leu-Thr-Lys-Gly-Pro-,
H-Gln-Gly-Ser-Phe-Leu-Thr-Lys-Gly-Pro-,
H-Asn-Gln-Gly-Ser-Phe-Leu-Thr-Lys-Gly-Pro-, or
H-Gly-Asn-Gln-Gly-Ser-Phe-Leu-Thr-Lys-Gly-Pro-, and
$R_2$ is -OH, -Gln-OH, -Gln-Gly-OH, -Gln-Gly-Asn-OH, or -Gln-Gly-Asn-Phe-OH, with the proviso that $R_1$ is not H-Thr-Lys-Gly-Pro- when $R_2$ is -Gln-Gly-Asn-Phe-OH, or a pharmaceutically acceptable salt thereof. Furthermore, pharmaceutical compositions are disclosed which contain said peptides.

EP 0 448 099 A2

The present invention relates to a novel peptide.

A living body protects itself against the invasion of bacteria or viruses, or internally-occurring diseases by utilizing the protective functions of an immune system, without which human beings could not survive for even one day.

Nevertheless, the immune system itself is the cause of some diseases. For example, autoimmune diseases or allergies are caused by the presence of the immune system in the living body. Further, hepatitis caused by the hepatitis virus occurs due to the presence of the immune system, and many other infective diseases are revealed through modification thereof by an immune reaction .

An immunodeficiency disease is believed to be due to a decline in the functional ability of the immune system. The immune function is exhibited by interaction of various immunocytes, and therefore, the immune system does not function properly, even if a defect occurs in only one of these immunocytes.

As mentioned above, the immune mechanism plays an important role in the maintaining of the living body from various aspects.

Many immunocytes are known, and many monoclonal antibodies recognizing the same are also known. For example, Leu3a and OKT4A exhibit a specific recognition of a helper T cell, because these monoclonal antibodies recognize the CD4 receptor on the surface of the helper T cell. Further, it is known that Leu3a and OKT4A inhibit infection by HIV (Human immunodeficiency virus) through CD4 receptor (Q. J. Sattentau et al., Science 234, 1120-1123, 1986). Furthermore, B. A. Jameson et al., Science 240, 1335-1339, 1988, have suggested as an epitope of OKT4A, a peptide consisting of 16 amino acids, i.e., CD4(32-47).

Taking into account the content of the report by Sattentau et al. in the article mentioned above, that OKT4A and Leu3a show a mutual cross-inhibition, the inventor of the present invention conducted research in an attempt to find an epitope of Leu3a.

First, a fragment peptide (having 19 amino acids) including the peptide CD4(32-47) having 16 amino acids proposed by Jameson et al. was synthesized and an examination of a competitive inhibitory activity for a T cell between the fragment peptide and Leu3a was carried out, but no such inhibitory activity was found.

Accordingly, various peptides other than said fragment peptide were synthesized and the inhibitory activity thereof was examined in the same manner. Surprisingly, it was found that a certain novel peptide exhibits such an inhibitory activity. Further, the minimum unit thereof exhibiting this inhibitory activity was identified and it was found that a substitution of one or more D-amino acids for a part of the amino acids constituting the novel peptide enhances the stability thereof in blood while maintaining the required inhibitory activity.

Accordingly, an object of the present invention is to provide a novel peptide having a physiological activity.

The present invention relates to a peptide having the general formula (I) (SEQ ID NO:1):

$$R_1\text{-Ser-Lys-Leu-Asn-Asp-Arg-Ala-Asp-Ser-Arg-Arg-Ser-Leu-Trp-Asp-}R_2 \quad (I)$$

wherein $R_1$ is H-, H-Pro-, H-Gly-Pro, H-Lys-Gly-Pro-, H-Thr-Lys-Gly-Pro-, H-Leu-Thr-Lys-Gly-Pro-, H-Phe-Leu-Thr-Lys-Gly-Pro-, H-Ser-Phe-Leu-Thr-Lys-Gly-Pro-,
H-Gly-Ser-Phe-Leu-Thr-Lys-Gly-Pro-,
H-Gln-Gly-Ser-Phe-Leu-Thr-Lys-Gly-Pro-,
H-Asn-Gln-Gly-Ser-Phe-Leu-Thr-Lys-Gly-Pro-, or
H-Gly-Asn-Gln-Gly-Ser-Phe-Leu-Thr-Lys-Gly-Pro-, and
$R_2$ is -OH, -Gln-OH, -Gln-Gly-OH, -Gln-Gly-Asn-OH, or -Gln-Gly-Asn-Phe-OH, with the proviso that $R_1$ is not H-Thr-Lys-Gly-Pro- when $R_2$ is -Gln-Gly-Asn-Phe-OH,
or a pharmaceutically acceptable salt thereof.

Unless otherwise indicated, the term "amino acid" used herein means L-amino acid. However, "L-" is optionally used as a prefix to the symbols of the amino acids if the amino acid in question should be indicated as an L-form. On the other hand, when the amino acid in question is a D-amino acid, "D-" is always used as a prefix to the symbols of the amino acids, to explicitly indicate same, except in the case of glycine. In the present specification, the sequence of the amino acids is expressed by placing N-terminus at the left and the C-terminus at the right, as usual in this field.

The abbreviations of the amino acids, the residues thereof, the peptides, the protecting groups, the reagents used, or the like, as used herein are in accordance with those formulated by the IUPAC-IUB

Committee on Biochemical Nomenclature Recommendation. Further, abbreviations commonly used in this field are also employed, as shown by the following Examples:

| | |
|---|---|
| Ala: | alanine, |
| Asn: | asparagine, |
| Cys: | cysteine, |
| Thr: | threonine, |
| Phe: | phenylalanine, |
| Pro: | proline, |
| Gly: | glycine, |
| Arg: | arginine, |
| Asp: | aspartic acid, |
| Ser: | serine, |
| Lys: | lysine, |
| Trp: | tryptophan, |
| Gln: | glutamine, |
| Leu: | leucine, |
| Ile: | isoleucine, |
| BOP: | benzotriazole-1-yl-oxy-tris-(dimethylamino)phosphonium-hexafluorophosphate, |
| Boc: | t-butoxycarbonyl, |
| Fmoc: | 9-fluorenylmethoxycarbonyl, |
| DIPCDI: | diisopropylcarbodiimide, |
| DCC: | dicyclohexylcarbodiimide, |
| $Bu^t$: | t-butyl, |
| Mtr: | 4-methoxy-2,3,6-trimetylbenzenesulphonyl, |
| OPfp: | pentafluorophenylester, |
| HOBt: | 1-hydroxybenzotriazole, |
| $OBu^t$:: | t-butylester, |
| DMF: | dimethylformamide. |

The peptide of the general formula (I) according to the present invention comprises 15 to 30 L-amino acids, but the peptide of the general formula (I) wherein $R_1$ is a hydrogen atom and $R_2$ is OH group, namely, the peptide comprising 15 L-amino acids and having the formula (II) (SEQ ID NO: 2):

H-Ser-Lys-Leu-Asn-Asp-Arg-Ala-Asp-Ser-Arg-Arg
-Ser-Leu-Trp-Asp-OH (II),

is preferred.

The present invention also relates to peptides wherein at least one of the amino acids constituting the peptide of the general formula (I) is substituted by a D-amino acid. The substituted peptide will be optionally referred to as an L/D peptide hereinafter. The preferred L/D peptides are those wherein one to four L-amino acids constituting the peptide of the formula (II) are substituted by one or more D-amino acids. Examples of these L/D peptides are as follows:

H-Ser-D-Lys-Leu-Asn-Asp-Arg-D-Ala-Asp-Ser-Arg-Arg-Ser-
Leu-Trp-Asp-OH (SEQ ID NO: 3) (IIIa)

H-Ser-D-Lys-Leu-Asn-Asp-Arg-D-Ala-Asp-Ser-Arg-D-Arg-Ser-
Leu-Trp-Asp-OH (SEQ ID NO: 4) (IIIb)

H-Ser-D-Lys-Leu-Asn-Asp-Arg-D-Ala-Asp-Ser-Arg-Arg-Ser-D-Leu-Trp-Asp-OH (SEQ ID NO: 5) (IIIc)

H-Ser-Lys-Leu-Asn-Asp-Arg-D-Ala-Asp-Ser-Arg-Arg-Ser-Leu-Trp-Asp-OH (SEQ ID NO: 6) (IIId)

H-Ser-Lys-Leu-Asn-Asp-Arg-D-Ala-Asp-D-Ser-Arg-Arg-Ser-Leu-Trp-Asp-OH (SEQ ID NO: 7) (IIIe)

H-Ser-Lys-Leu-Asn-Asp-Arg-D-Ala-Asp-Ser-D-Arg-Arg-Ser-Leu-Trp-Asp-OH (SEQ ID NO: 8) (IIIf)

H-Ser-Lys-Leu-Asn-Asp-Arg-D-Ala-Asp-Ser-Arg-D-Arg-Ser-Leu-Trp-Asp-OH (SEQ ID NO: 9) (IIIg)

H-Ser-Lys-Leu-D-Asn-Asp-Arg-D-Ala-Asp-Ser-Arg-Arg-Ser-Leu-Trp-Asp-OH (SEQ ID NO: 10) (IIIh)

H-Ser-Lys-Leu-Asn-Asp-Arg-Ala-D-Asp-Ser-Arg-Arg-Ser-Leu-Trp-Asp-OH (SEQ ID NO: 11) (IIIi)

H-Ser-Lys-D-Leu-Asn-Asp-Arg-D-Ala-Asp-Ser-Arg-Arg-Ser-D-Leu-Trp-Asp-OH (SEQ ID NO: 12) (IIIj)

H-Ser-D-Lys-Leu-D-Asn-Asp-Arg-D-Ala-Asp-Ser-Arg-D-Arg-Ser-Leu-Trp-Asp-OH (SEQ ID NO: 13) (IIIk)

H-Ser-D-Lys-Leu-D-Asn-Asp-Arg-D-Ala-Asp-Ser-Arg-Arg-Ser-D-Leu-Trp-Asp-OH (SEQ ID NO: 14) (IIIl)

The peptides of the general formula (I) and the L/D peptides according to the present invention may be in the form of pharmaceutically acceptable salts thereof. The pharmaceutically acceptable salt is an acid addition salt or metallic complex, such as a complex with zinc, iron, calcium, magnesium, aluminum or the like. As examples of the acid addition salt, there may be mentioned hydrochloride, hydrobromide, sulfate, phosphate, tannate, oxalate, fumarate, gluconate, alginate, maleate, acetate, trifluoroacetate, citrate, benzoate, succinate, malate, ascorbate, tartrate or the like. Further, the salt may be a carboxylate, such as a salt with an alkaline metal (sodium or potassium salt, or the like) or an alkaline earth metal (calcium or magnesium salt, or the like), or ammonium salt.

The peptide of the general formula (I) according to the present invention can be prepared by condensing respective amino acids or lower peptides in the order of the amino acid sequence as shown in the formula (I), using a conventional method for synthesizing a peptide. The techniques of such a peptide synthesis are described, for example, in Seikagaku-Jikken-Koza 1, Tanpakushitsu-no-Kagaku IV, (Handbook for Experiments in Biochemistry 1, Chemistry of Protein IV), Tokyo-Kagaku-Dojin, 1978.

The synthesis of the peptides is commenced by attaching a first amino acid carrying an appropriately protected amino or carboxyl group to an inactive solid support (solid phase synthesis), or by dissolving such an amino acid in an appropriate solvent (liquid phase synthesis). After the protecting group in the first amino acid is eliminated, a second amino acid carrying a similarly protected amino or carboxyl group is bonded to the first amino acid. After all of the amino acids are successively bonded in a similar manner, the residual protecting groups and solid support are successively or simultaneously removed to obtain the desired peptide.

The peptide of the general formula (I) according to the present invention is preferably prepared by the solid phase synthesis (for example, Fmoc or Boc method), and various automatic synthesizers are commercially available, for example, Model 9600 or 9500 from Biosearch Inc., U.S.A., for the solid phase synthesis. The desired peptides may be prepared in accordance with a known protocol. For a detailed description of the above, refer to, for example, Solid Phase Peptide Synthesis 2nd Ed., Pierce Chemical

Company, 1984; or D. Hudson, J. Org. Chem. Vol. 53, pp. 617-624 (1988).

In the solid phase synthesis, various protecting groups may be used to block reactive sites of an amino acid precursor, and the selection of the protecting groups depends upon the anticipated cleavage conditions and properties of the final products.

In the Fmoc method, an amino acid carrying an α-amino group protected by 9-fluorenylmethoxycarbonyl (Fmoc) group is used as a reactant, and in the Boc method, an amino acid carrying an α-amino group protected by t-butyloxycarbonyl (Boc) group is reacted.

The deprotection of the Fmoc group is carried out by ammonolysis, but that of the Boc group is carried out by acidolysis.

More specifically, the ammonolysis of the Fmoc group is carried out, for example, by adding a solution (prepared by adding an organic amine, such as piperidine, to a 1:1-mixture of dimethylformamide and toluene), to the resin in an amount of 5 to 20 ml per 1 g of resin, and allowing a reaction therebetween at 0 to 40°C for 1 to 10 minutes. In this process, preferably the agitation is caused by an inert gas (for example, nitrogen or argon gas), mechanical shaking or the like, or a circulation of the liquid by a pump or the like.

The acidolysis of the Boc group is carried out, for example, by adding 5 to 20 ml (per 1 g of resin) of a trifluoroacetic acid solution in dichloromethane to the resin, and allowing a reaction therebetween at 0 to 40°C for 10 to 60 minutes. Preferably, to suppress side reactions, 1 to 5% anisole or the like is added. After the reaction is completed, excess trifluoroacetic acid is removed by adding a 20% organic amine (for example, diisopropylethylamine) solution in dichloromethane, in an amount of 5 to 20 ml (per 1 g of resin) in each case, 1 to 10 times. In this process, preferably, the agitation is caused by an inert gas (for example, nitrogen or argon gas), mechanical shaking or the like, or a circulation of the liquid by a pump or the like.

As examples of the supports used in the solid phase synthesis, there may be mentioned synthetic resins such as chloromethyl, oxymethyl, p-alkoxybenzylalcohol, PAM or PAC resin, or a composite resin such as polyamide-kieselguhr-resin. In the case of chloromethyl resin, a terminal amino acid may be introduced to the resin to form an amino acid resin, by adding an organic amine (for example, triethylamine), or using a salt of a protected amino acid (for example, potassium, cesium, or tetramethylammonium salt). In the case of other resins, a terminal amino acid is introduced by, for example, a DCC/DMAP, active ester or oxidation-reduction method.

Examples of the amino acids used as a reactant and having an activated carboxyl group are preferably symmetric acid anhydrides derived from DIPCDI (or DCC), DIPCDI (or, DCC)/HOBt, BOP/HOBt, or active esters, such as pentafluorophenylester, N-hydroxysuccinimide ester, or benzotriazole ester.

Among the symmetric acid anhydride methods, the BOP/HOBt or DIPCDI/HOBt method is preferred for the Fmoc group, and the DIPCDI method is preferred for the Boc group. In each case, preferably 1 to 20 equivalents (generally, 5 to 10 equivalents) of amino acid with respect to an amino acid resin are used.

More particularly, in the BOP/HOBt method, for example, an equivalent amount of 0.05 to 0.4M N-methylmorpholine solution in dimethylformamide or N-methylpyrrolidone is added to Fmoc-amino acid, BOP reagent (in an equivalent amount with respect to the Fmoc-amino acid) and HOBt (in an equivalent amount with respect to the Fmoc-amino acid) , and a reaction therebetween is carried out at 0 to 40°C for 1 to 10 minutes. In this process, preferably the agitation is caused by an inert gas (for example, nitrogen or argon gas) to completely dissolve the amino acid and the condensation agent. The resulting reaction mixture is immediately added to the amino acid resin or peptide resin, and a condensation reaction is carried out at 0 to 40°C for 0.5 to 3 hours. In this process, preferably the agitation is caused by an inert gas (for example, nitrogen or argon gas), mechanical shaking or the like, or a circulation of the liquid by a pump or the like.

In the DIPCDI/HOBt method, for example, a solution of Fmoc-amino acid and HOBt [which has been dissolved in dimethylformamide or N-methylpyrrolidone so that the concentration of HOBt in an equivalent amount to that of the Fmoc-amino acid is 0.2 to 0.5M] and a DIPCDI solution having an equimolar concentration in dichloromethane are added one after the other in the same amounts of 0.1 to 0.5 ml at 0 to 40°C, admixed together, and then reacted for 1 to 10 minutes. The resulting reaction mixture is immediately added to the amino acid resin or peptide resin, and a condensation reaction is carried out at 0 to 40°C for 0.5 to 3 hours. In this process, preferably the agitation is caused by an inert gas (for example, nitrogen or argon gas), mechanical shaking or the like, or a circulation of the liquid by a pump or the like.

Asparagine is preferably condensed by the active ester method, because the symmetric acid anhydride method causes dehydration reaction of amide side chains. As the active ester in the Fmoc method, OPfp is preferred. More particularly, Fmoc-Asn-OPfp and 1.5 equivalent of HOBt are dissolved in dimethylformamide or N-methylpyrrolidone so that the concentrations thereof are 0.1 to 0.5M, and added to the amino acid resin or peptide resin: The subsequent steps are substantially the same as those used in the above process.

In the DIPCDI method, for example, a solution of Boc-amino acid [which has been dissolved in

dimethylformamide or N-methylpyrrolidone so that the concentration thereof is 0.2 to 0.5M], and DIPCDI solution having an equimolar concentration in dichloromethane are added one after the other in the same amounts of 0.1 to 0.5 ml at 0 to 40 °C, admixed, and reacted for 1 to 10 minutes. The resulting reaction mixture is immediately added to the amino acid resin or peptide resin, and a condensation reaction is carried out at 0 to 40 °C for 0.5 to 3 hours. In this process, preferably the agitation is caused by an inert gas (for example, nitrogen or argon gas), mechanical shaking or the like, or a circulation of the liquid by a pump or the like.

In the Fmoc method, examples of the protecting groups for reactive cites in side chains are the t-butyl group (for Ser or Thr), t-butylester (for Asp), t-butoxycarbonyl (for Lys), 4-methoxy-2,3,6-trimethylbenzenesulfonyl group (for Arg), or 4,4'-methoxybenzhydryl or 2,4,6-trimethoxybenzyl group (for Asn or Gln). The protecting groups used in the Boc method are, for example, the tosyl group (for Arg), benzylester group (for Asp), O-benzyl group (for Ser or Thr), or chlorobenzyloxycarbonyl group (for Lys).

The selection of methods for eliminating the protecting groups and releasing the synthesized peptides from the support resins is made in accordance with the properties of the protecting groups and the synthesized peptides.

In the Fmoc method, trifluoroacetic acid is usually used, but other organic acids, such as 70% trifluoromethanesulfonic acid in trifluoroacetic acid, and hydrogen bromide, or a mixture of trifluoromethanesulfonic acid, trifluoroacetic acid and dichloromethane, may be used. The trifluoroacetic acid can further contain a scavenger, such as 1,2-ethanedithiol, anisole and/or thioanisole. Further, trimethylsilylbromide may be added to completely remove the Mtr group of the arginine side chain. After cleavage and deprotection, the resin is filtered off, trifluoroacetic acid is evaporated, and ethylether is added to precipitate the peptide. The peptide is then filtered and purified by usual isolation and purification procedures, such as column chromatography or high performance liquid chromatography, and the resulting peptide may be lyophilized and stored.

In the Boc method, the deprotection of the protecting groups in the side chains and cleavage of the peptide from the resin may be carried out simultaneously, for example, by adding hydrogen fluoride at -20 to 0 °C. In this method, a scavenger, such as anisole, thioanisole, p-thiocresol or dimethylsulfide may be used at the same time.

The L/D peptide according to the present invention may be prepared by the method for preparing the peptide having the general formula (I), except that only one or more L-amino acids to be substituted are replaced with one or more D-amino acids.

The physiological activity of the peptide of the general formula (I) according to the present invention is observed when the inhibitory activity thereof is examined by an enzyme-immunoassay (EIA), using Leu3a (anti-CD4 monoclonal antibody) and a membrane fraction of a human T cell line (for example, MOLT4 cell, or CCRF-CEM cell).

The L/D peptide is useful as a physiological agent, because of an enhanced stability in blood while maintaining the above inhibitory activity.

Further, the peptide of the general formula (I) and the L/D peptide is expected to exhibit physiological activities, particularly an anti-autoimmunity and an inhibitory action on transplantation rejection reactions (graft rejection).

## Examples

The present invention now will be further illustrated by, but is by no means limited to, the following Examples.

### Method for Determining Inhibitory Activity

In the following Examples, the inhibitory activity of the synthesized peptides was determined by the following EIA method.

MOLT4 cells (2 x $10^6$) are suspended in 2 ml of 40mM HEPES buffer (pH 7.4) containing 1mM EGTA [ethyleneglycol-bis($\beta$-aminoethylether)-N,N,N',N'-tetraacetic acid], 1mM PMSF (phenylmethylsulfonylfluoride), 1000 KIU/ml aprotinin and 150mM sodium chloride, and twice treated for 15 seconds at dial power of 1, by a ultrasonic homogenizer (Sonifier 250; Branson Ultrasonic Co.). Then, 5 ml of 50mM bicarbonate buffer (pH 9.6) is added to the treated liquid, centrifugation (100 x g, 5 minutes) is carried out, and the supernatant is taken out and centrifuged (15000 x g, 10 minutes). Then, 1 ml of 50mM bicarbonate buffer is added to the resulting precipitate and treated three times under the same conditions as above by the ultrasonic homogenizer. After 1 ml of 50mM bicarbonate buffer is further added, the

resulting suspension is poured into a 96-well microtiter plate at an amount of 50 μl/well, and the plate is incubated overnight at 4° C. After the suspension liquid is removed, 50 μl/well of 10mM phosphate buffered saline (PBS) containing 0.2 glutaraldehyde is added, and the plate is allowed to stand at room temperature for 3 minutes to ensure fixation. After the reaction liquid is removed, the plate is washed 5 times by 10mM PBS, 350 μl/well of 10mM PBS containing 2% bovine serum albumin (BSA) is added, and the plate is allowed to stand for 1 hour for blocking. After the reaction liquid is removed, the plate is washed 5 times by BPBS (10mM PBS containing 0.2% BSA).

Then, the peptide to be tested is dissolved in BPBS in various concentrations, and added to each well in an amount of 50 μl/well, and at the same time, 25 μl/well of a solution [prepared by diluting biotin-labelled Leu3a by BPBS in a concentration of 600 ng/ml] is added. The plate is incubated overnight at 4° C, and then washed 8 times with BPBS containing 0.05% Tween 20. Thereafter, 50 μl/well of a solution [prepared by adding 90 μl of the reagent A (avidin DH) of ABC kit (Vector Laboratories Inc.) and 90 μl of the reagent B (biotinylated horseradish peroxidase H) of the same kit to 5 ml of BPBS containing 0.05% Tween 20] is added, and incubated at room temperature for 4 hours. The plate is then washed 8 times with 20mM imidazole buffered saline containing 0.02% Tween 20, and a mixture of equal amounts of a substrate solution A (ABTS reagent) and a substrate solution B (aqueous hydrogen peroxide) of Hybri-Clonal EIA Screening Kit (Kirkegaard & Perry Laboratories Inc.) is added to each well in an amount of 100 μl/well. After allowing to stand for 1 hour at room temperature, for coloring, the absorbance at 405 nm is measured by a microplate reader (MPR-A4: Tosoh Co., Ltd.).

A binding rate of the biotin-labelled Leu3a is calculated from the absorbance, and an amount of peptide which inhibits the binding by 50% is defined as $IC_{50}$ (μg/well).

Example 1:

**Synthesis of H-Ser-Lys-Leu-Asn-Asp-Arg-Ala-Asp-Ser-Arg-Arg-Ser-Leu-Trp-Asp-OH (SEQ ID NO: 2) (II)**

First, 500 mg of Fmoc-Asp($OBu^t$)-O-polymer (p-alkoxybenzylalcohol resin; 0.62 mmol/g) were added to the reaction vessel R2 in a Biosearch Model 9600 peptide synthesizer. Amino acid derivatives, the BOP reagent, and HOBt were added to the amino acid vessels 1 to 14 in the amounts shown in Table 1, respectively. Thereafter solvents were added to the solvent vessels 33 to 35, 50 to 51 and 53 to 55 in the amounts shown in Table 2, respectively, and the synthesis was commenced in accordance with the Program FMOCBOP. The subroutines 1 used in the program for the elimination reactions of the protecting groups from the amino acids and the subroutines 2 used therein for the condensation reactions are also listed in Table 1. The abbreviations used in Table 1 are as follows:

Subroutine 1:
FBDBK2M: Elimination of Fmoc group and subsequent activation of amino acid for 2 minutes.
FBDBK3M: Elimination of Fmoc group and subsequent activation of amino acid for 3 minutes.
FBDBKARG: Elimination of Fmoc group and subsequent activation of Arg (option for Arg) *.
FBDBKASN: Elimination of Fmoc group and subsequent dissolution of Asn active ester.

Subroutine 2:
BOP-1 x 1: Coupling for 1 hour.
BOP-1 x 1A: Coupling for 1 hour (option for Arg) *.
BOP-1 x 2: Coupling for 2 hour.

After the reaction was completed, the resulting peptide resin was recovered from R2, and dried under a reduced pressure, 4.0 ml of trifluoroacetic acid, 0.6 ml of thioanisole, 0.3 ml of 1,2-ethanedithiol, and 0.1 ml of anisole were added to 500 mg of the peptide resin, and the whole was stirred at room temperature for 1 hour. Then, 0.7 ml of trimethylsilylbromide was added while cooling on ice, and stirred for 2 hours while

(* The program was modified, because of the low solubility of Arg.)

cooling on ice. The resin was then filtered, trifluoroacetic acid was evaporated, absolute ethylether was added to obtain a precipitate, and the resulting precipitate was filtered and dried. The precipitate (100 mg) was dissolved by adding 6 ml of 0.1% aqueous trifluoroacetic acid solution and 1.5 ml of acetonitrile, and the solution was purified by D-ODS-5 column (inner diameter 20 mm x length 250 mm; Yamamura Chemical Laboratories Co., Ltd.) for a reverse-phase partition chromatography, under a linear gradient condition (from 20:80 to 60:40) between a 0.1% aqueous trifluoroacetic acid solution and a 0.1% trifluoroacetic acid solution in acetonitrile. After the solvent was removed, 10 mg of the residue was again dissolved in 5 ml of a 0.1% aqueous trifluoroacetic acid solution, and then, a further purification was carried out, using the same column and solvents, except that the gradient condition was changed to from 20:80 to 40:60. After acetonitrile was evaporated, the residue was lyophilized to obtain the trifluoroacetic acid salt of the desired peptide (II). An optical rotation $[\alpha]_D^{26}$(C=0.2, 1M acetic acid), $Rf^I$ value [1-butanol-pyridine-acetic acid-water (15:3:10:12)] and $Rf^{II}$ value [1-propanol-pyridine-acetic acid-water (10:5:4:4)] of a thin layer chromatography, and the inhibitory activity, were measured of the resulting peptide, and the results were as shown in Table 3.

## Table 1: Amino Acids Used in Synthesis

| No. | Amino acid derivative | Amount (g) | BOP (g) | HOBt (g) | Subroutine 1 | Subroutine 2 |
|---|---|---|---|---|---|---|
| 1 | Fmoc-L-Trp-OH | 0.85 | 0.884 | 0.306 | FBDBK2M | BOP-1X1 |
| 2 | Fmoc-L-Leu-OH | 0.71 | 0.884 | 0.306 | FBDBK3M | BOP-1X1 |
| 3 | Fmoc-L-Ser($Bu^t$)-OH | 0.78 | 0.884 | 0.306 | FBDBK2M | BOP-1X1 |
| 4 | Fmoc-L-Arg(Mtr)-OH | 1.24 | 0.884 | 0.306 | FBDBKARG | BOP-1X1A |

| | | | | | | |
|---|---|---|---|---|---|---|
| 5 | Fmoc-L-Arg(Mtr)-OH | 1.24 | 0.884 | 0.306 | FBDBKARG | BOP-1X1A |
| 6 | Fmoc-L-Ser($Bu^t$)-OH | 0.78 | 0.884 | 0.306 | FBDBK2M | BOP-1X1 |
| 7 | Fmoc-L-Asp($OBu^t$)-OH | 0.82 | 0.884 | 0.306 | FBDBK2M | BOP-1X1 |
| 8 | Fmoc-L-Ala-OH | 0.62 | 0.884 | 0.306 | FBDBK2M | BOP-1X1 |
| 9 | Fmoc-L-Arg(Mtr)-OH | 1.24 | 0.884 | 0.306 | FBDBKARG | BOP-1X1A |
| 10 | Fmoc-L-Asp($OBu^t$)-OH | 0.82 | 0.884 | 0.306 | FBDBK2M | BOP-1X1 |
| 11 | Fmoc-L-Asn-OPfp | 1.04 | 0 | 0.46* | FBDBKASN | BOP-1X2 |
| 12 | Fmoc-L-Leu-OH | 0.71 | 0.884 | 0.306 | FBDBK3M | BOP-1X1 |
| 13 | Fmoc-L-Lys(Boc)-OH | 0.94 | 0.884 | 0.306 | FBDBK2M | BOP-1X1 |
| 14 | Fmoc-L-Ser($Bu^t$)-OH | 0.78 | 0.884 | 0.306 | FBDBK2M | BOP-1X1 |
| | Number of moles (mmole) | 2 | 2 | 2(*=3) | | |

Table 2: Solvents Used in Synthesis

| Solvent vessel No. | Solvent |
|---|---|
| 55 | dimethylformamide:dichloromethane = 2200 : 2200 ml |
| 54 | dichloromethane = 300 ml |
| 53 | dimethylformamide = 300 ml |
| 51 | piperidine:dimethylformamide:toluene = 180 : 210 : 210 ml |
| 50 | methanol = 200 ml |
| 35 | dimethylformamide:dichloromethane = 400 : 400 ml |
| 34 | dimethylformamide = 300 ml |
| 33 | N-methylmorpholine:dimethylformamide = 5.5 : 244.5 ml |

The trifluoroacetic acid salts of the following four peptides (Ia), and (1) to (3) were synthesized by a method similar to the program of the automated method for synthesizing the above peptide (II), and the optical rotation, $Rf^{I}$ value, $Rf^{II}$ value, and inhibitory activity thereof were measured, respectively. The results are shown in Table 3.

Peptide (Ia) consisting of 30 amino acids, according to the present invention:

H-Gly-Asn-Gln-Gly-Ser-Phe-Leu-Thr-Lys-Gly-Pro-Ser-Lys-Leu-Asn-Asp-Arg-Ala-Asp-Ser-Arg-Arg-Ser-Leu-Trp-Asp-Gln-Gly-Asn-Phe-OH

Comparative peptide (1) consisting of 14 amino acids:

H-Lys-Leu-Asn-Asp-Arg-Ala-Asp-Ser-Arg-Arg-Ser-Leu-Trp-Asp-OH

Comparative peptide (2) consisting of 14 amino acids:

H-Ser-Lys-Leu-Asn-Asp-Arg-Ala-Asp-Ser-Arg-Arg-Ser-Leu-Trp-OH

Comparative peptide (3) consisting of 19 amino acids:

H-Lys-Asn-Ser-Asn-Gln-Ile-Lys-Ile-Leu-Gly-Asn-Gln-Gly-Ser-Phe-Leu-Thr-Lys-Gly-OH

[The peptide (3) includes the CD4 (32-47) fragment peptide consisting of 16 amino acids and disclosed in B. A. Jameson, et al, Science, 240, 1335-1339, 1988.]

Table 3

| Peptide | Optical rotation $[\alpha]_D^{26}$ | $Rf^I$ | $Rf^{II}$ | Inhibitory activity $IC_{50}$ μg/well |
|---|---|---|---|---|
| II | −43.50 | 0.53 | 0.40 | 10 |
| Ia | −62.71(3) | 0.66 | 0.25 | 4 |
| 1 | −30.50(2) | 0.60 | 0.17 | >500 |
| 2 | −42.00(2) | 0.60 | 0.20 | >500 |
| 3 | −49.00(1) | 0.74 | 0.31 | 500 |

(1) measured at 25°C (2) measured at 24°C

(3) measured at 28°C, C=0.7, 1M acetic acid.

As apparent from Table 3, an elimination of the N-terminal Ser from the peptide (II) of the present invention results in a reduction of the inhibitory activity thereof, and an elimination of the C-terminal Asp results in a considerable reduction of the inhibitory activity. Therefore, it is obvious that the peptide consisting of 15 amino acids is a minimum unit at which an activity is exhibited.

Example 2:

Preparation of peptides including substituted D- amino acid(s) (L/D peptides)

The trifluoroacetic acid salts of the following L/D peptides of the formulae (IIIa) to (IIIi) according to the present invention were synthesized, as in Example 1.

H-Ser-D-Lys-Leu-Asn-Asp-Arg-D-Ala-Asp-Ser-Arg-Arg-Ser-Leu-Trp-Asp-OH (SEQ ID NO: 3) (IIIa)

H-Ser-D-Lys-Leu-Asn-Asp-Arg-D-Ala-Asp-Ser-Arg-D-Arg-Ser-Leu-Trp-Asp-OH (SEQ ID NO: 4) (IIIb)

H-Ser-D-Lys-Leu-Asn-Asp-Arg-D-Ala-Asp-Ser-Arg-Arg-Ser-D-Leu-Trp-Asp-OH (SEQ ID NO: 5) (IIIc)

H-Ser-Lys-Leu-Asn-Asp-Arg-D-Ala-Asp-Ser-Arg-Arg-Ser-Leu-Trp-Asp-OH (SEQ ID NO: 6) (IIId)

H-Ser-Lys-Leu-Asn-Asp-Arg-D-Ala-Asp-D-Ser-Arg-Arg-Ser-Leu-Trp-Asp-OH (SEQ ID NO: 7) (IIIe)

H-Ser-Lys-Leu-Asn-Asp-Arg-D-Ala-Asp-Ser-D-Arg-Arg-Ser-Leu-Trp-Asp-OH (SEQ ID NO: 8) (IIIf)

H-Ser-Lys-Leu-Asn-Asp-Arg-D-Ala-Asp-Ser-Arg-D-Arg-Ser-Leu-Trp-Asp-OH (SEQ ID NO: 9) (IIIg)

H-Ser-Lys-Leu-D-Asn-Asp-Arg-D-Ala-Asp-Ser-Arg-Arg-Ser-Leu-Trp-Asp-OH (SEQ ID NO: 10) (IIIh)

H-Ser-Lys-Leu-Asn-Asp-Arg-Ala-D-Asp-Ser-Arg-Arg-Ser-Leu-Trp-Asp-OH (SEQ ID NO: 11) (IIIi)

The physicochemical data and inhibitory activity of the trifluoroacetic acid salts of the L/D peptides are shown in Table 4.

Table 4

| Peptide | Optical rotation $[\alpha]_D^{26}$ | $Rf^I$ | $Rf^{II}$ | Inhibitory activity $IC_{50}$µg/well |
|---|---|---|---|---|
| IIIa | −37.50(2) | 0.55 | 0.43 | 36 |
| IIIb | −30.00(2) | 0.54 | 0.42 | 25 |
| IIIc | −25.50 | 0.57 | 0.47 | 22 |
| IIId | −37.50(3) | 0.53 | 0.40 | 32 |
| IIIe | −42.50(2) | 0.52 | 0.40 | 10 |
| IIIf | −30.00 | 0.52 | 0.40 | 36 |
| IIIg | −30.76(1)(3) | 0.53 | 0.40 | 23 |
| IIIh | −28.50(2) | 0.52 | 0.39 | 60 |
| IIIi | −47.50 | 0.51 | 0.39 | 56 |

(1) C=0.13; (2) measured at 28°C; (3) measured at 27°C

Example 3: Stability of L/D Peptides

(3-1) Synthesis of Fmoc-Ser-D-Lys-Leu-Asn-Asp-Arg-D-Ala-Asp-Ser-Arg-D-Arg-Ser-Leu-Trp-Asp-OH (Fmoc-IIIb)

To the reaction vessel R2 of a Biosearch Model 9600 peptide synthesizer were added 500 mg of Fmoc-Asp(OBu$^t$)-O-polymer (p-alkoxybenzylalcohol resin; 0.62 mmol/g), and amino acid derivatives, the BOP reagent, and HOBt were added to the amino acid vessels 1 to 14 in the amounts shown in Table 1, respectively, except that the amino acids No. 4, No. 8, and No. 13 were replaced by D-amino acids. Solvents were added to the solvent vessels 33 to 35, 50 to 51 and 53 to 55 in the amounts shown in Table 2, respectively, and the synthesis was commenced in accordance with the Program FMOCBOPA (program of deprotecting Fmoc group at an N-terminal amino acid was omitted). The subroutines 1 used in the program for the deprotection reactions of the protecting groups of the amino acids and the subroutines 2 used therein for the condensation reactions were the same as those shown in Table 1.

After the reaction was completed, the resulting peptide resin was recovered from R2, and dried under a reduced pressure, 4 ml of trifluoroacetic acid, 0.6 ml of thioanisole, 0.3 ml of 1,2-ethanedithiol, and 0.1 ml of anisole were added to 500 mg of the peptide resin, and the whole was stirred at room temperature for 1.5 hours. Then, 0.7 ml of trimethylsilylbromide was added while cooling on ice, and stirred for 2 hours while cooling on ice. The resin was filtered, trifluoroacetic acid and trimethylsilylbromide were evaporated, an absolute ethylether was added to obtain a precipitate, and the resulting precipitate was filtered, and dried. The precipitate was purified as in Example 1 to obtain the trifluoroacetate of the title L/D peptide.

Using the same procedure, a trifluoroacetic acid salt of a peptide not containing D-amino acid and having the formula

Fmoc-Ser-Lys-Leu-Asn-Asp-Arg-Ala-Asp-Ser-Arg-Arg-Ser-Leu-Trp-Asp-OH (Fmoc-II)

was synthesized.

(3-2) Test for Stability in Blood

A PBS solution (0.5 ml) containing 50 μg/ml of the trifluoroacetic acid salt of the peptide having an N-terminal Fmoc group [i.e., (Fmoc-II)] or the trifluoroacetic acid salt of the L/D peptide having an N-terminal Fmoc group [i.e., (Fmoc-IIb)] prepared in the above (3-1) was thoroughly admixed with 0.5 ml of normal human serum, and incubated at 37 ± 1 °C for 1, 2, 4, 6, 12, 18, and 24 hours. After the reaction was completed, the reaction liquid was adsorbed to Sep-Pak C18 cartridge (Waters Inc.), and then the cartridge was washed with 2 ml of PBS and eluted with 2 ml of 50% aqueous acetonitrile solution containing 0.1% trifluoroacetic acid. After the acetonitrile was evaporated, the residue was lyophilized, and dissolved in 1 ml of 0.1% aqueous trifluoroacetic acid solution. An amount of non-degraded peptides was analyzed, using an analyzing ODS column (AP-303/S-5: inner diameter 4.6 mm x length 250 mm: Yamamura Chemical Laboratories Co.), and the survival rates at various times were calculated, on the basis that the rate at 0 hour is 100%. A time at which the rate is 50% is designated as a half-life. The results are shown in Table 5.

Table 5: Half-life of Peptides in Serum

| Sample | Half-life (Hr) | Increasing Ratio of Half-life due to Substitution by D-amino acid(s) |
|---|---|---|
| Fmoc-II | 0.5 | 1 |
| Fmoc-IIIb | 2 | 4 |

The peptide according to the present invention comprises 15 to 30 amino acids, i.e., is a relatively low molecular peptide, and thus has good handling properties upon administration or the like. The L/D peptide according to the present invention is stable in blood while maintaining an inhibitory activity, and thus may exhibit a sufficient physiological activity in a living body.

## SEQUENCE LISTING

1. SEQ ID NO: 1:

SEQUENCE TYPE: amino acid
SEQUENCE LENGTH: 17
TOPOLOGY: linear
MOLECULE TYPE: peptide
PROPERTIES: Leu3a epitope

Xaa Ser Lys Leu Asn Asp Arg Ala Asp Ser Arg Arg Ser Leu Trp
5 10 15
Asp Xaa

Xaa, Position 1 ($R_1$):
- H,
- H-Pro,
- H-Gly-Pro,
- H-Lys-Gly-Pro-,
- H-Thr-Lys-Gly-Pro-,
- H-Leu-Thr-Lys-Gly-Pro-,
- H-Phe-Leu-Thr-Lys-Gly-Pro-,
- H-Ser-Phe-Leu-Thr-Lys-Gly-Pro-,
- H-Gly-Ser-Phe-Leu-Thr-Lys-Gly-Pro-,
- H-Gln-Gly-Ser-Phe-Leu-Thr-Lys-Gly-Pro-,
- H-Asn-Gln-Gly-Ser-Phe-Leu-Thr-Lys-Gly-Pro- or
- H-Gly-Asn-Gln-Gly-Ser-Phe-Leu-Thr-Lys-Gly-Pro-

Xaa, Position 17 ($R_2$):
- -OH,
- -Gln-OH,
- -Gln-Gly-OH,
- -Gln-Gly-Asn-OH or
- -Gln-Gly-Asn-Phe-OH

2. <u>SEQ ID NO: 2:</u>

SEQUENCE TYPE: amino acid
SEQUENCE LENGTH: 15
TOPOLOGY: linear
MOLECULE TYPE: peptide
PROPERTIES: Leu3a epitope

Ser Lys Leu Asn Asp Arg Ala Asp Ser Arg Arg Ser Leu Trp Asp
5 10 15

3. <u>SEQ ID NO: 3:</u>

SEQUENCE TYPE: amino acid
SEQUENCE LENGTH: 15
TOPOLOGY: linear
MOLECULE TYPE: peptide
PROPERTIES: Leu3a epitope

Ser Xaa Leu Asn Asp Arg Xaa Asp Ser Arg Arg Ser Leu Trp Asp
5 10 15

Xaa, Position 2: D-Lys
Xaa, Position 7: D-Ala

4. <u>SEQ ID NO: 4:</u>

SEQUENCE TYPE: amino acid
SEQUENCE LENGTH: 15
TOPOLOGY: linear
MOLECULE TYPE: peptide
PROPERTIES: Leu3a epitope

Ser Xaa Leu Asn Asp Arg Xaa Asp Ser Arg Xaa Ser Leu Trp Asp
5 10 15

Xaa, Position 2: D-Lys
Xaa, Position 7: D-Ala

Xaa, Position 2: D-Lys
Xaa, Position 7: D-Ala
Xaa, Position 11: D-Arg

5. <u>SEQ ID NO: 5:</u>

SEQUENCE TYPE: amino acid
SEQUENCE LENGTH: 15
TOPOLOGY: linear
MOLECULE TYPE: peptide
PROPERTIES: Leu3a epitope

Ser Xaa Leu Asn Asp Arg Xaa Asp Ser Arg Arg Ser Xaa Trp Asp
5 10 15

Xaa, Position 2: D-Lys
Xaa, Position 7: D-Ala
Xaa, Position 13: D-Leu

6. <u>SEQ ID NO: 6:</u>

SEQUENCE TYPE: amino acid
SEQUENCE LENGTH: 15
TOPOLOGY: linear
MOLECULE TYPE: peptide
PROPERTIES: Leu3a epitope

Ser Lys Leu Asn Asp Arg Xaa Asp Ser Arg Arg Ser Leu Trp Asp
5 10 15

Xaa, Position 7: D-Ala

7. <u>SEQ ID NO: 7:</u>

SEQUENCE TYPE: amino acid
SEQUENCE LENGTH: 15
TOPOLOGY: linear

MOLECULE TYPE: peptide
PROPERTIES: Leu3a epitope

Ser Lys Leu Asn Asp Arg Xaa Asp Xaa Arg Arg Ser Leu Trp Asp
5 10 15

Xaa, Position 7: D-Ala
Xaa, Position 9: D-Ser

8. SEQ ID NO: 8:

SEQUENCE TYPE: amino acid
SEQUENCE LENGTH: 15
TOPOLOGY: linear
MOLECULE TYPE: peptide
PROPERTIES: Leu3a epitope

Ser Lys Leu Asn Asp Arg Xaa Asp Ser Xaa Arg Ser Leu Trp Asp
5 10 15

Xaa, Position 7: D-Ala
Xaa, Position 10: D-Arg

9. SEQ ID NO: 9:

SEQUENCE TYPE: amino acid
SEQUENCE LENGTH: 15
TOPOLOGY: linear
MOLECULE TYPE: peptide
PROPERTIES: Leu3a epitope

Ser Lys Leu Asn Asp Arg Xaa Asp Ser Arg Xaa Ser Leu Trp Asp
5 10 15

Xaa, Position 7: D-Ala
Xaa, Position 11: D-Arg

10. <u>SEQ ID NO: 10:</u>

| | |
|---|---|
| SEQUENCE TYPE: | amino acid |
| SEQUENCE LENGTH: | 15 |
| TOPOLOGY: | linear |
| MOLECULE TYPE: | peptide |
| PROPERTIES: | Leu3a epitope |

Ser Lys Leu Xaa Asp Arg Xaa Asp Ser Arg Arg Ser Leu Trp Asp
5 10 15

Xaa, Position 4: D-Asn
Xaa, Position 7: D-Ala

11. <u>SEQ ID NO: 11:</u>

| | |
|---|---|
| SEQUENCE TYPE: | amino acid |
| SEQUENCE LENGTH: | 15 |
| TOPOLOGY: | linear |
| MOLECULE TYPE: | peptide |
| PROPERTIES: | Leu3a epitope |

Ser Lys Leu Asn Asp Arg Ala Xaa Ser Arg Arg Ser Leu Trp Asp
5 10 15

Xaa, Position 8: D-Asp

12. <u>SEQ ID NO: 12:</u>

| | |
|---|---|
| SEQUENCE TYPE: | amino acid |
| SEQUENCE LENGTH: | 15 |
| TOPOLOGY: | linear |
| MOLECULE TYPE: | peptide |
| PROPERTIES: | Leu3a epitope |

Ser Lys Xaa Asn Asp Arg Xaa Asp Ser Arg Arg Ser Xaa Trp Asp
5 10 15

Xaa, Position 3: D-Leu
Xaa, Position 7: D-Ala
Xaa, Position 13: D-Leu

13. <u>SEQ ID NO: 13:</u>

| | |
|---|---|
| SEQUENCE TYPE: | amino acid |
| SEQUENCE LENGTH: | 15 |
| TOPOLOGY: | linear |
| MOLECULE TYPE: | peptide |
| PROPERTIES: | Leu3a epitope |

```
Ser Xaa Leu Xaa Asp Arg Xaa Asp Ser Arg Xaa Ser Leu Trp Asp
                 5                  10                  15
```

Xaa, Position 2: D-Lys
Xaa, Position 4: D-Asn
Xaa, Position 7: D-Ala
Xaa, Position 11: D-Arg

14. <u>SEQ ID NO: 14:</u>

| | |
|---|---|
| SEQUENCE TYPE: | amino acid |
| SEQUENCE LENGTH: | 15 |
| TOPOLOGY: | linear |
| MOLECULE TYPE: | peptide |
| PROPERTIES: | Leu3a epitope |

```
Ser Xaa Leu Xaa Asp Arg Xaa Asp Ser Arg Arg Ser Xaa Trp Asp
                 5                  10                  15
```

Xaa, Position 2: D-Lys
Xaa, Position 4: D-Asn
Xaa, Position 7: D-Ala
Xaa, Position 13: D-Leu

## Claims

1. A peptide having the general formula (I) (SEQ ID NO: 1):

$$R_1\text{-Ser-Lys-Leu-Asn-Asp-Arg-Ala-Asp-Ser-Arg-Arg-Ser-Leu-Trp-Asp-}R_2 \qquad (I)$$

wherein $R_1$ is H-, H-Pro-, H-Gly-Pro, H-Lys-Gly-Pro-, H-Thr-Lys-Gly-Pro-, H-Leu-Thr-Lys-Gly-Pro-, H-Phe-Leu-Thr-Lys-Gly-Pro-, H-Ser-Phe-Leu-Thr-Lys-Gly-Pro-,
H-Gly-Ser-Phe-Leu-Thr-Lys-Gly-Pro-,
H-Gln-Gly-Ser-Phe-Leu-Thr-Lys-Gly-Pro-,
H-Asn-Gln-Gly-Ser-Phe-Leu-Thr-Lys-Gly-Pro-, or
H-Gly-Asn-Gln-Gly-Ser-Phe-Leu-Thr-Lys-Gly-Pro-, and
$R_2$ is -OH, -Gln-OH, -Gln-Gly-OH, -Gln-Gly-Asn-OH, or -Gln-Gly-Asn-Phe-OH, with the proviso that $R_1$ is not H-Thr-Lys-Gly-Pro- when $R_2$ is -Gln-Gly-Asn-Phe-OH, or a pharmaceutically acceptable salt thereof.

2. A peptide according to claim 1, having the formula (II) (SEQ ID NO: 2):

$$\text{H-Ser-Lys-Leu-Asn-Asp-Arg-Ala-Asp-Ser-Arg-Arg-Ser-Leu-Trp-Asp-OH} \qquad (II),$$

or a pharmaceutically acceptable salt thereof.

3. A peptide having a structure wherein at least one of the amino acids constituting the peptide of the general formula (I) set forth in claim 1 or of the general formula (II) set forth in claim 2 is substituted by a D-amino acid, or a pharmaceutically acceptable salt thereof.

4. A peptide according to claim 3, wherein one to four amino acids constituting the peptide are substituted by a D-amino acid, or a pharmaceutically acceptable salt thereof.

5. A peptide according to claim 3 or 4, having the formula represented in SEQ ID NO: 3:

H-Ser-D-Lys-Leu-Asn-Asp-Arg-D-Ala-Asp-Ser-Arg-Arg-Ser-Leu-Trp-Asp-OH,

or a pharmaceutically acceptable salt thereof.

6. A peptide according to claim 3 or 4, having the formula represented in SEQ ID NO: 4:

H-Ser-D-Lys-Leu-Asn-Asp-Arg-D-Ala-Asp-Ser-Arg-D-Arg-Ser-Leu-Trp-Asp-OH,

or a pharmaceutically acceptable salt thereof.

7. A peptide according to claim 3 or 4, having the formula represented in SEQ ID NO: 5:

H-Ser-D-Lys-Leu-Asn-Asp-Arg-D-Ala-Asp-Ser-Arg-Arg-Ser-D-Leu-Trp-Asp-OH,

or a pharmaceutically acceptable salt thereof.

**8.** A peptide according to claim 3 or 4, having the formula represented in SEQ ID NO: 6:

H-Ser-Lys-Leu-Asn-Asp-Arg-D-Ala-Asp-Ser-Arg-Arg-Ser-Leu-Trp-Asp-OH,

or a pharmaceutically acceptable salt thereof.

**9.** A peptide according to claim 3 or 4, having the formula represented in SEQ ID NO: 7:

H-Ser-Lys-Leu-Asn-Asp-Arg-D-Ala-Asp-D-Ser-Arg-Arg-Ser-Leu-Trp-Asp-OH,

or a pharmaceutically acceptable salt thereof.

**10.** A peptide according to claim 3 or 4, having the formula represented in SEQ ID NO: 8:

H-Ser-Lys-Leu-Asn-Asp-Arg-D-Ala-Asp-Ser-D-Arg-Arg-Ser-Leu-Trp-Asp-OH,

or a pharmaceutically acceptable salt thereof.

**11.** A peptide according to claim 3 or 4, having the formula represented in SEQ ID NO: 9:

H-Ser-Lys-Leu-Asn-Asp-Arg-D-Ala-Asp-Ser-Arg-D-Arg-Ser-Leu-

Trp-Asp-OH,

or a pharmaceutically acceptable salt thereof.

**12.** A peptide according to claim 3 or 4, having the formula represented in SEQ ID NO: 10:

**H-Ser-Lys-Leu-D-Asn-Asp-Arg-D-Ala-Asp-Ser-Arg-Arg-Ser-Leu-Trp-Asp-OH,**

or a pharmaceutically acceptable salt thereof.

**13.** A peptide according to claim 3 or 4, having the formula represented in SEQ ID NO: 11:

H-Ser-Lys-Leu-Asn-Asp-Arg-Ala-D-Asp-Ser-Arg-Arg-Ser-Leu-Trp-Asp-OH,

or a pharmaceutically acceptable salt thereof.

**14.** A peptide according to claim 3 or 4, having the formula represented in SEQ ID NO: 12:

H-Ser-Lys-D-Leu-Asn-Asp-Arg-D-Ala-Asp-Ser-Arg-Arg-Ser-D-Leu-Trp-Asp-OH,

or a pharmaceutically acceptable salt thereof.

**15.** A peptide according to claim 3 or 4, having the formula represented in SEQ ID NO: 13:

H-Ser-D-Lys-Leu-D-Asn-Asp-Arg-D-Ala-Asp-Ser-Arg-D-Arg-Ser-Leu-Trp-Asp-OH,

or a pharmaceutically acceptable salt thereof.

**16.** A peptide according to claim 3 or 4, having the formula represented in SEQ ID NO: 14:

H-Ser-D-Lys-Leu-D-Asn-Asp-Arg-D-Ala-Asp-Ser-Arg-Arg-Ser-D-Leu-Trp-Asp-OH,

or a pharmaceutically acceptable salt thereof.

**17.** Pharmaceutical composition containing a peptide according to any one of claims 1 to 16.

**18.** Pharmaceutical composition according to claim 17 for the treatment of auto-immune diseases.

**19.** Pharmaceutical composition according to claim 17 for the treatment of transplantation rejection reactions.